(19) Europäisches Patentamt
European Patent Office
Office européen des brevets

(11) **EP 1 884 772 A1**

(12) **EUROPEAN PATENT APPLICATION**

(43) Date of publication:
**06.02.2008 Bulletin 2008/06**

(51) Int Cl.:
**G01N 33/00** (2006.01)          **F01N 3/20** (2006.01)

(21) Application number: **06076520.3**

(22) Date of filing: **02.08.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **Nederlandse Organisatie voor Toegepast-Natuurwetenschappelijk Onderzoek TNO
2628 VK  Delft (NL)**

(72) Inventors:
• **van Genderen, Mathijs**
  **2497 NV Den Haag (NL)**
• **van Aken, Marcus Gerardus**
  **4007 SZ  Tiel (NL)**

(74) Representative: **van Loon, C.J.J. et al**
**c/o VEREENIGDE
Johan de Wittlaan 7
2517 JR  Den Haag (NL)**

(54) **Quality control of selective catalytic reduction reagents**

(57)     The invention is directed to a method for monitoring the quality of selective catalytic reduction agents, in particular for monitoring the quality of $NH_3$ producing reagents, such as urea solutions.

The method of the present invention allows for determining the equivalent $NH_3$ concentration of any selective catalytic reduction reagent that forms $NH_3$ upon heating. Subsequently, the amount of injected reagent can be adjusted to the measured equivalent $NH_3$ concentration in order to optimise the $NO_x$ reduction.

process chamber

a set volume is pressed

AdBlu

**Figure 1 Dosing concept 1**

EP 1 884 772 A1

**Description**

The invention is directed to a method for monitoring the quality of selective catalytic reduction agents, in particular for monitoring the quality of $NH_3$ producing reagents, such as urea solutions.

In order to make fuel powered engines, particularly diesel engines, less harmful for the environment, new technologies are being developed for reducing harmful emissions. Especially diesel engines are responsible for high amounts of $NO_x$. Most technologies for reducing these emissions are based on after treatment of the exhaust gas.

A promising technique for reducing $NO_x$ emissions is the so-called Selective Catalytic Reduction (SCR) technique. The fundamental approach to this technology is to inject ammonia or urea into the exhaust gas to convert $NO_x$ into nitrogen and water. This conversion is often achieved with the aid of a catalyst. Urea is selected over ammonia since ammonia is poisonous and difficult to handle. Urea can be decomposed into $NH_3$ according to the following reactions.

$$(NH_2)_2CO \xrightarrow{\Delta T} NH_3 + HNCO$$

$$HNCO + H_2O \longrightarrow NH_3 + CO_2$$

Subsequently $NO_x$ from the exhaust gas is reduced by $NH_3$ so that $N_2$ and $H_2O$ are formed which are harmless for the environment. This reduction of $NO_x$ can take place in the presence of oxygen. The following reactions are assumed to take place.

(1) $6NO + 4NH_3 \rightarrow 5N_2 + 6H_2O$

(2) $4NO + 4NH_3 + O_2 \rightarrow 4N_2 + 6H_2O$

(3) $6NO_2 + 8NH_3 \rightarrow 7N_2 + 12H_2O$

(4) $2NO_2 + 4NH_3 + O_2 \rightarrow 3N_2 + 6H_2O$

(5) $NO + NH_2 + 2NH_3 \rightarrow 2N_2 + 3H_2O$

Reaction (2) being the dominant reaction under normal conditions, whereas reaction (5) is the more preferred reaction. The extra $NO_2$ that is required for reaction (5) may be obtained by using a diesel oxidation catalyst.

From these reaction equations it follows that the amount of injected urea should be well controlled. If the amount of injected urea is too low, then not all $NO_x$ is converted and as a result the exhaust gas will comprise $NO_x$. On the other hand, an excess of injected urea leads to the formation and emission of dangerous $NH_3$.

A standard selective catalytic reduction reagent that is applied in SCR systems is AdBlue®, which is a 32.5 wt. % aqueous urea solution. However, other selective catalytic reduction reagents with different urea concentrations are also known. Furthermore, selective catalytic reduction reagents have been developed which have a freezing point that is lower than the freezing point of AdBlue® which is -11 °C. For example, Denoxium™ involves a mixture of urea with ammonium formate, thereby lowering the freezing point to -30 °C. Since all these different selective catalytic reduction reagents have a different equivalent $NH_3$ concentration, they require a different dosing in the exhaust in order to have optimal catalytic $NO_x$ reduction.

The present inventors further believe that they are the first to have realised that although injecting reducing agents into the exhaust gas stream may have beneficial effects for the environment, which may be a good ground to enforce the use of systems employing injection of reducing agents by legislation, in practice such systems can be tampered with or can even be made completely dysfunctional. This type of abuse is not entirely hypothetical, because the user of the engine generally will have to pay for keeping the stock of reducing agents on a proper level without seeing any direct benefit for it. Abuse may for instance involve using water instead of urea solution, by which the concentration of urea will lower considerably or even become zero. This will not be noted easily by visual inspection. If SCR is to be enforced successfully, it is important to have a reliable system of monitoring the quality of selective catalytic reduction agents.

WO-A-00/75643 describes a method for adjusting the amount of injected urea in order to have optimal $NO_x$

conversion. The method is based on a fluorescently tagged selective catalytic reduction reagent by which the equivalent concentration of $NH_3$ can be determined. Disadvantages of this method include that the fluorescent tracer has to be applied to the selective catalytic reduction reagent in a pretreatment and that the mentioned organic fluorescent tracers are sensitive to high temperatures.

Object of the present invention is to provide a method for monitoring the quality of selective catalytic reduction agents, which at least partly overcomes the above-mentioned shortcomings and disadvantages.

In accordance with the present invention, this objective is met by a monitoring the quality of selective catalytic reduction agents. Accordingly, in a first aspect the present invention is directed to a method for monitoring the quality of selective catalytic reduction agents comprising determining the equivalent $NH_3$ concentration of the selective catalytic reduction reagent by carrying out the subsequent steps of:

- injecting a known amount of the selective catalytic reduction reagent into a chamber;
- converting the amount of reagent in the chamber to form $NH_3$; and
- measuring the amount of $NH_3$ with an $NH_3$ sensor.

The method of the present invention allows for determining the equivalent $NH_3$ concentration of any selective catalytic reduction reagent that forms $NH_3$ by pyrolysis and/or hydrolysis. Subsequently, the amount of injected reagent can be adjusted to the measured equivalent $NH_3$ concentration in order to optimise the $NO_x$ reduction.

Advantageously, the selective catalytic reduction reagent does not need to be subjected to a pretreatment, but can be applied directly in the tank.

Preferably the selective catalytic reduction reagent comprises urea.

According to the invention a small amount of the selective catalytic reduction reagent is injected into a small process chamber. It is advantageous when the amount of reagent is fixed so that during each batch the same amount of reagent is converted in the chamber. This is for instance possible by pressing a fixed volume from a container that is filled with selective catalytic reduction reagent. The volume can be directly pressed into the process chamber.

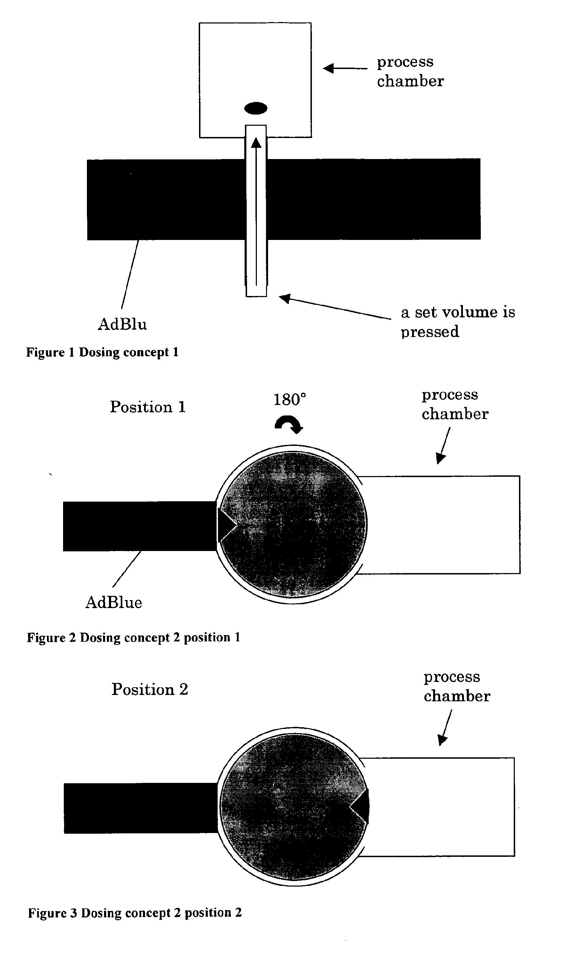

Fig. 1-3 schematically depict suitable configurations for injecting fixed amounts of reagents into the process chamber. In the embodiment of Fig. 1 this is obtained by using a piston moving through a cylinder filled with reagent.

Injecting a fixed amount of selective catalytic reduction reagent into the process chamber can also be obtained by providing a circular body with a notch in a closed shell connecting a container filled with selective catalytic reduction reagent with the process chamber. This embodiment is depicted in Fig. 2 and 3. When the notch is turned to the container, the notch is filled with a fixed amount of reagent. If subsequently the circular body is turned so that the notch faces the process chamber, the fixed amount of selective catalytic reduction agent is released into the process chamber.

Of course, also other systems can be designed in order to inject a fix volume of selective catalytic reduction reagent into the process chamber. The amount of reagent that is injected into the chamber can vary and depends on factors such as the size of the process chamber, the detection range of the $NH_3$ sensor, the type of vehicle (*e.g.* heavy duty (trucks), medium duty (vans) or light duty (cars)), *etc*. The invention can also be applied in non-road engines, *e.g.* bulldozers, shovels, generators and the like. For light duty engines, the size of the chamber is preferably smaller than 1 $cm^3$, typically from 0.01 to 0.5 $cm^3$. For heavy duty engines, the size may be as large as 1 $dm^3$ for instance from 0.1 to 0.5 $dm^3$.

The lower limit of the injected amount is in also determined by the sensitivity of the $NH_3$ sensor.

Table 1 list, by way of example, different process chamber volumes and typical detection limits for the $NH_3$ sensor. These dimensions and concentrations can be used in accordance with the present invention. The required volume of reagent solution (*i.e.* aqueous 32.5 wt.% AdBlue solution) and the corresponding droplet diameter is indicated as well.

**Table 1**

| Process Chamber Volume | NH₃ Sensor upper limit (volume %) | Injected Volume | Droplet Diameter |
|---|---|---|---|
| 1 $dm^3$ | 10% | 0.384 mL | 9.02 mm |
| 1 $dm^3$ | 10000 ppm | 0.0384 mL | 4.18 mm |
| 1 $dm^3$ | 1000 ppm | 3.84 nL | 1.94 mm |
| 1 $dm^3$ | 100 ppm | 0.384 nL | 0.902 mm |
| 1 $cm^3$ | 10% | 0.384 nL | 0.902 mm |
| 1 $cm^3$ | 10000 ppm | 0.0384 nL | 0.418 mm |

In the process chamber, the selective catalytic reduction reagent is converted into $NH_3$ and $CO_2$. At a temperature of below 360 °C, HNCO can be formed as a by-product when urea is decomposed. Furthermore, if urea is slowly heated unwanted polymerisation may occur. Therefore, if no catalyst to assist urea decomposition is used, it is preferred that the injected amount of selective catalytic reduction reagent is brought to a temperature of more than 360 °C as quickly as possible. This can for example be achieved by flash evaporating the injected volume into the chamber at a temperature of above 360 °C. For example, the fixed volume can be brought to a pressure which is higher than the pressure in the chamber before it is injected. At temperatures above 360 °C, HNCO that may have been formed is also converted to $NH_3$ and $CO_2$. Preferably the temperature in the process chamber is more than 400 °C in the absence of a catalyst.

In order to achieve a complete conversion of HNCO at low temperatures, in particular below 360 °C, a hydrolysis catalyst may be used, such as zeolite or vanadium.

To improve the conversion of the SCR reagent, a solid catalyst can be applied in the reaction chamber, such as precious metal based catalysts and/or zeolite or alumina based catalysts, or combinations thereof.

The conversions in the process chamber can be carried out at pressures that are just above atmospheric pressure, *e.g.* from about 1 to 1.1 atm.

Any type of $NH_3$ sensor may be used that is compatible with the size of the chamber. It is preferred that the sensor is selective for $NH_3$, because otherwise errors in the equivalent $NH_3$ concentrations may occur. The $NH_3$ sensor may for example be a solid-state $NH_3$ sensor, a semiconductor $NH_3$ sensor, an electrochemical $NH_3$ sensor, a field effect $NH_3$ sensor, a $NO_x$ sensor which is used for detecting $NH_3$ by cross selectivity, or any other known selective $NH_3$ sensor in the art. If the sensor is heat sensitive (for example certain solid state, semiconductor and electrochemical sensors), the formed gasses can be moved into a separate chamber where they can cool down in order to be measured.

After the fixed amount of selective catalytic reduction reagent has been converted in the process chamber and the $NH_3$ concentration has been assessed, the chamber can be cleaned so that no residue influences the following batch. This can be done by flushing a gas, such as air or exhaust gas, through the process chamber at an elevated pressure. The flushing gas may be present in a second large chamber where the gas is brought under pressure by heating. Subsequently, a valve in the process chamber is opened so that gasses can leave the process chamber. Then the large chamber and the process chamber are connected so that the flushing gas under pressure can enter the process chamber and clean it.

The effluent from the process chamber may be fed into the exhaust gas stream, where the $NH_3$ can react with NOx in the conventional manner.

The method of the invention can be carried out at any point in time, *e.g.* directly after starting the engine, or a multitude of times while the engine is running. Typical sampling frequencies may vary widely, *e.g.* a single comparison check can be made by measuring once before and once after starting the engine. Alternatively, the frequency may be several samples per hour or minute. If actual control of the flow of reduction agents is desired, typical frequencies may be from 0.1 to 10 $s^{-1}$, *e.g.* 1 $s^{-1}$.

In another embodiment, the measured equivalent $NH_3$ concentration of the selective catalytic reduction reagent is used to adjust the amount of reagent that is injected into the exhaust for reducing the $NO_x$ emission. This can be done by means of a standard algorithm in which the measured equivalent $NH_3$ concentration is compared to the equivalent $NH_3$ concentration of a standard urea solution, such as AdBlue®, and the injection amount is calculated accordingly. Then, the calculated injection amount can be compared with the actual injection amount and the injection can be adjusted so as to optimise the amount of selective catalytic reduction reagent to the reduction of $NO_x$. This can for instance be achieved by the method described in WO-A-02/33232.

## Claims

1. A method for monitoring the quality of selective catalytic reduction agents used in NOx reduction of exhaust gases, comprising determining the equivalent $NH_3$ concentration of the selective catalytic reduction reagent by carrying out the subsequent steps of:

   - injecting a known amount of the selective catalytic reduction reagent into a chamber;
   - converting the amount of reagent in the chamber to form $NH_3$; and
   - measuring the amount of $NH_3$ with an $NH_3$ sensor.

2. A method according to claim 1, followed by a step wherein the chamber is purged.

3. A method according to any of the preceding claims, further comprising a step wherein the equivalent $NH_3$ concentration of the selective catalytic reduction agent is used to adjust the amount of the selective catalytic reduction agent which is injected into the exhaust gas stream.

4. A method according to any of the preceding claims, wherein the injection of the selective reduction reagent into the chamber is a flash evaporation.

5. A method according to any of the preceding claims, wherein the catalytic reduction reagent comprises urea.

6. A method according to any of the preceding claims, wherein the step of measuring the amount of $NH_3$ formed is carried out directly after starting the engine, or a multitude of times while the engine is running.

7. A method according to any of the previous claims, wherein the $NH_3$ formed in said chamber is injected in said exhaust gas.

8. A method according to any of the preceding claims, wherein the temperature of the chamber is more than 360 °C, preferably more than 400 °C.

process
chamber

AdBlu

a set volume is
pressed

**Figure 1 Dosing concept 1**

Position 1     180°     process
chamber

AdBlue

**Figure 2 Dosing concept 2 position 1**

Position 2     process
chamber

**Figure 3 Dosing concept 2 position 2**

**European Patent Office**    **EUROPEAN SEARCH REPORT**

Application Number

EP 06 07 6520

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 968 464 A (PETER-HOBLYN JEREMY D ET AL) 19 October 1999 (1999-10-19)<br>* abstract; figures 2,3 *<br>* column 5, lines 16-25 *<br>* column 7, line 55 - column 8, line 3 *<br>----- | 1-8 | INV.<br>G01N33/00<br><br>ADD.<br>F01N3/20 |
| X | US 6 679 051 B1 (VAN NIEUWSTADT MICHIEL J ET AL) 20 January 2004 (2004-01-20)<br>* abstract; figures 1A,1B,5A,5B *<br>* column 3, lines 44-63 *<br>* column 4, lines 17-39 *<br>----- | 1-8 | |
| X | US 2003/033799 A1 (SCHEYING GERD) 20 February 2003 (2003-02-20)<br>* abstract; figure 1 *<br>* paragraphs [0039], [0042] *<br>* paragraphs [0055], [0056] *<br>----- | 1-8 | |
| X | US 5 540 047 A (DAHLHEIM CHRISTIAN ET AL) 30 July 1996 (1996-07-30)<br>* abstract; figures 1,13,14 *<br>----- | 1-8 | |
| | | | TECHNICAL FIELDS SEARCHED (IPC) |
| X | MOOS R ET AL: "Selective ammonia exhaust gas sensor for automotive applications" SENSORS AND ACTUATORS B, ELSEVIER SEQUOIA S.A., LAUSANNE, CH, vol. 83, no. 1-3, 15 March 2002 (2002-03-15), pages 181-189, XP004344503 ISSN: 0925-4005<br>* abstract; figure 1 *<br>* page 181, left-hand column *<br>----- | 1-8 | G01N<br>B01D<br>F01N |

The present search report has been drawn up for all claims

| Place of search | Date of completion of the search | Examiner |
|---|---|---|
| Munich | 14 February 2007 | Hanisch, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 07 6520

This annex lists the patent family members relating to the patent documents cited in the above-mentioned European search report.
The members are as contained in the European Patent Office EDP file on
The European Patent Office is in no way liable for these particulars which are merely given for the purpose of information.

14-02-2007

| Patent document cited in search report | | Publication date | Patent family member(s) | | Publication date |
|---|---|---|---|---|---|
| US 5968464 | A | 19-10-1999 | US | 6203770 B1 | 20-03-2001 |
| US 6679051 | B1 | 20-01-2004 | DE | 10328839 A1 | 26-02-2004 |
| | | | GB | 2393140 A | 24-03-2004 |
| | | | JP | 2004169683 A | 17-06-2004 |
| | | | US | 2004103653 A1 | 03-06-2004 |
| US 2003033799 | A1 | 20-02-2003 | DE | 10139142 A1 | 20-02-2003 |
| | | | EP | 1283332 A2 | 12-02-2003 |
| US 5540047 | A | 30-07-1996 | DE | 4334071 C1 | 09-02-1995 |
| | | | EP | 0653237 A1 | 17-05-1995 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0075643 A
- WO 0233232 A